# EUROPEAN PATENT APPLICATION

(11) **EP 1 074 249 A1**
(43) Date of publication of application: **07.02.2001**
(21) Application number: 99114237.3
(22) Date of filing: 27.07.1999
(51) Int. Cl.: A61K 9/36, A61K 9/46, A61K 9/24

(54) **A pharmaceutical tablet system for releasing at least one active substance during a release period subsequent to a no-release period**

(71) Applicant: JAGOTEC AG, 4132 Muttenz (CH)
(72) Inventor: Chopinet-Cote, Sandrine, 68110 Ilzach (FR); Grenier, Dr.Pascal, 68510 Kappelen (FR); Quinton, Jacques, F-68640 Waldighofen (FR); Vergnault, Dr.Guy, F-68300 St.Louis (FR); Zimmer, Dr.Robert, F-68100 Mulhouse (FR); Ricchi, Elisabeth, F-68100 Mulhouse (FR)
(74) Representative: Köver, François

(57) **Abstract**

The tablet system has a core-centred body covered with at least one couple of adjacent layers, one element fully enclosing the other. The enclosed element contains active substance and will, upon exposure to aqueous medium, undergo complete degradation during a period of release of active substance. The enclosing element is devoid of active substance and formulated to delay the latter's release from the enclosed element by a no-release period. To this effect, it has an intrinsic porosity that will remain constant while allowing and controlling the penetration of the aqueous medium towards the underlying enclosed element whose alteration will in turn cause a fragmentation of the enclosing element that will allow the aqueous medium to directly reach the enclosed element and provide for rapid establishment and progress to completion of the release of active substance. The layers may be made by spray-coating and/or pressing.

## Description

### TECHNICAL FIELD OF THE INVENTION

The present invention relates to a tablet system for pharmaceutical use capable, when contacted with an aqueous medium, of releasing at least one active substance during a release period of predetermined duration subsequent to a no-release period of predetermined duration, the tablet system having a core-centred body comprised of at least one couple of tablet elements adjacent to each other, one of which is fully enclosed within the other one, thus to define an enclosing tablet element and an enclosed tablet element, the enclosed element being comprised of active substance and formulated to release the active substance upon exposure to the aqueous medium while undergoing essentially complete degradation during the release period, the enclosing element being devoid of active substance and formulated to delay the release of the active substance from the enclosed element by the no-release period.

### BACKGROUND OF THE INVENTION

Tablet systems for pharmaceutical use capable of releasing an active substance during a release period of predetermined duration have been developed to reduce the number of times a corresponding drug has to be administered to a patient, as well as to improve the patient's compliance to the administration of the drug. Such controlled-release systems are beneficial to the patient in that they allow to maintain a desired level of active substance in the patient's plasma for a desired time.

Further developments of such controlled-release systems allow to release predetermined quantities of active substances in succession at respective predetermined release rates. Depending on the particular embodiment, system can also be devised to provide two release periods subsequent to one another that are separated from each other by a no-release period, each period having a respective predetermined duration.

To provide a no-release period of predetermined duration preceding the onset of a release period, the prior art approach has been to enclose a layer or core that is comprised of the active substance within a retarding layer that is devoid of active substance. The disappearance or destruction of the retarding layer by erosion, dissolution and/or digestion or the like will take some time, causing the release of the active substance to be delayed by the desired time lag.

One prior art approach to provide the desired time lag is disclosed e.g. in US-A-4871549. In this kind of system, a core or inner layer that is comprised of the active substance is enclosed within an intermediate layer that is comprised or devoid of active substance and capable of swelling, and this intermediate layer is enclosed within an outer layer that is insoluble and devoid of active substance. When the system is placed in the gastro-intestinal tract, the intermediate layer is induced to swell, eventually causing the outer layer to burst after a desired time lag, thus allowing the active substance to be released. In such a system, the thickness of the outer layer controls the diffusion rate of water to the intermediate layer and thus, the time lag is controlled both by the thickness of the insoluble outer layer and the force generated by the intermediate layer when swelling. To prepare a multiparticulate system of this kind involves a long and complex process with at least two steps for each population of pellets. Moreover, to compress the pellets into a tablet is liable to disrupt the retarding layer, eventually causing early release of the active substance. For instance, the tablet described in Example 10 of US-A-4871549 is formulated with some 20 % by weight of a swelling desintegrant (sodium carboxymethylcellulose) that helps to provide for explosion of the outer membrane: such a concentration of swelling desintegrant will cause the core to swell to such an extent that a matrix will be formed in which the active substance will have to move by diffusion for being released. As no in vitro performances of this system are disclosed, it remains at issue whether a fast release of active substance can be obtained.

Thus, tentative improvements of the above system have been proposed that are disclosed e.g. in US-A-5464633 and US-4933186 which describe tablets having a core comprised of the active substance that is enclosed within a retarding layer devoid of active substance. This retarding layer is able to form a gel that will dissolve slowly to leave, after a predetermined period of time, the core exposed to the dissolution medium. A fast release of active substance will take place when the retarding layer is completely dissolved, however, prior to the complete dissolution of the retarding layer the active substance will be able to diffuse through the gel barrier and give rise to a slow release.

Further tentative improvements of the above systems have been proposed that are disclosed e.g. in US-A-5472708, US-A-5364620, US-A-5840329, US-A-5529790, US-A-5397574, US-A-5837284 and WO-A-99/18938 which describe systems whose retarding layers are comprised, in combination, of a water insoluble and permeable polymer as well as one or both of a water soluble polymer and a permeability modulating agent, which improves control of the diffusion through the retarding layer. However, where a water soluble or water permeable polymer is used in the retarding layer, as soon as body fluids act on the layer this will produce micropores or channels that allow the underlying layer to release active substance in a sustained manner and may also weaken the retarding layer, which may lead to the latter's rupture after an uncontrolled period of time. The system described in WO-A-99/18938 aims at obviating these effects apparently to obtain a better control, however, a small variation of the quantity of retarding layer film-coated onto the core entails a large variation of the no-release period or lag time duration. As shown more particularly in Example 1 and Fig. 1 of WO-A-99/18938, to increase the retarding layer from 17 mg to 20 mg i.e. by 3 mg (which represents but a 1.2 percent increase of the final tablet weight) will lead to a 3 hours increase of the lag time duration. Hence, the system described in WO-A-99/18938 is extremely sensitive to a parameter, the quantity of retarding layer film-coated onto the core, which it is delicate to control strictly in the course of the spraying process. Moreover, the retarding layer of the above mentioned systems has a complex structure with at least two components in its formulation and hence, this retarding layer is not continuous, which adds to the fragility of the systems.

Generally, it appears that in most of the above mentioned types of tablet system, when a no-release period is desired, the longer the time lag the longer the subsequent duration of the release of the active substance, which can be appreciated for instance in the disclosure of US-A-4933186 (cf. more particularly in the drawings) and/or US-A-5464633 (cf. more particularly in the Tables VII and VIII). By way of example, whereas the disclosure of US-A-5840329 shows a fast release that follows a delay of about 4 hours it may not be inferred therefrom that a similarly fast release could be obtained after a longer delay.

In this context, any release - whether first or subsequent - is commonly termed "rapid" or "prompt" or "short" or "fast" when 80 % of the release takes place within one hour or less of the onset of the release, irrespective of any delay possibly applied to this onset; any of the above terms "rapid" or "prompt" or "short" or "fast" shall generally be construed herein in, or similar to, the aforesaid manner.

Still further tentative improvements of the above systems have been proposed that are disclosed e.g. in US-A-5629017 which describes a system involving a tablet comprised of one couple of tablet elements adjacent to each other, namely a core and a coating layer. The fully enclosed core is comprised of the active substance and formulated to release the latter upon exposure to the aqueous medium while undergoing essentially complete degradation. The coating layer is devoid of active substance and formulated to delay the release of the latter. Optionally, an enteric coating also devoid of active substance can be provided to enclose the aforesaid system. The delay-providing mechanism is said (cf. US-A-5629017, col. 7, lines 25-39) not to be well understood, however, apparently "not to depend on chemical or biochemical reactions" but rather on "slow and uniform dispersion" of the coating layer "by physical interaction" with the aqueous medium. This amounts to have a coating layer that experiences erosion, dissolution and/or digestion but through which the aqueous medium is not allowed to penetrate, consistent with the fatty composition of the coating layer, the presence therein of a surfactant that will promote its erosion, dissolution and/or digestion, the stated "direct functional" relationship between the thickness of the coating layer and the duration of the resulting no-release period, and the statement that other properties of the material such as the melting point of the fatty components have less influence. Accordingly, in such a tablet system a no-release period of long duration can only be obtained by providing a thick coating layer, which results in poor economic efficiency for lack of moderate use of the material. Moreover, it is well known in the art that the quality of a fatty compound is liable to poor reproducibility from batch to batch because of polymorphism, especially for natural fatty compounds. Hence, the performance of systems such as that which is disclosed in US-A-5629017 is liable to be poorly reproducible, due to their formulation comprised of fatty compounds. Besides, to assess the economic efficiency of such systems it must be taken into consideration that the process of spraying fatty compounds is delicate to perform, as is well known in the art.

Still other systems have been proposed that have planar layers superposed in a stack that is enclosed within a coating in such manner that an outer face of an outer layer of the stack is left uncovered and unprotected by the coating and thus, will eventually be exposed to body fluids (it readily appears that such systems are not constructed as a multi-layered body comprised in succession of a core and a plurality of layers, each of which provides a coating completely enclosing the immediately underlying layer). Examples of such embodiments are disclosed e.g. in US-A-5213808, US-A-5487901 and EP-A-0788790. The US-A-5487901 and EP-A-0788790 systems and some embodiments of the US-A-5213808 system have a three-layered stack construction that can provide at most two release periods separated by one no-release period.

Also - and more particularly for the system disclosed in EP-A-0788790 - as these systems are only partly enclosed any poor contact and attachment between the stack of layers and the coating will allow fluids to infiltrate the system, causing construction fragility as well as undesirable variations more particularly of the in vivo release rate of the active substance from the innermost i.e. lowermost layer of the stack, e.g. the so-called "dose dumping".

Furthermore - and more particularly for the system disclosed in US-A-5487901 - after the upper layer of the stack has been dissolved the coating is liable to collapse onto the intermediate layer of the stack and then prevent the body fluids from accessing properly the next two layers in succession. The intermediate layer will fail to be eroded and/or gelified and/or dissolved properly, eventually impairing the action of the body fluids on the lowermost layer comprised of the active substance and causing undesirable variability of the system.

The complex stack construction impairs the reliability of this system and involves complex and delicate manufacturing procedures supported on an appreciable know-how, all of this being very costly and in contradiction with the requirements of large scale or mass production.

Some other embodiments of the US-A-5213808 system are still more complex and thus, still more costly and unadapted to large scale or mass production, in particular due to the use of polymers subject to specific melting constraints that are poorly compatible with standard manufacturing requirements.

Referring to the prior art systems of the kind mentioned above in which a tablet system for pharmaceutical use is capable, when contacted with an aqueous medium, of releasing one or more active substances during a respective release period of respective predetermined duration subsequent to a respective no-release period of predetermined duration, none of these systems discloses a no-release period that would exceed by far about 8 hours - in such systems, a longer time lag appears to remain beyond reach except for the system disclosed in WO-A-99/18938, however, in each of whose Examples the long lag time duration corresponds to the in vitro analysis of only one tablet, which lets the reproducibility remain at issue. Yet, a time lag of about 6 hours or more is desirable in that it allows to release the active substance in the patient's colon region. Considering that drug absorption in the patient's colon region proceeds at a poor rate, the active substance released there should be released fast, which excludes the use of prior art systems having a retarding layer that does not burst - as said above about such prior art systems, the longer the time lag the longer the subsequent duration of the release of the active substance.

### SUMMARY OF THE INVENTION

The present invention aims at improving the release and time lag properties of tablet systems for pharmaceutical use of the kind recited in the preamble.

More particularly, the present invention aims at providing tablet systems for pharmaceutical use of the kind recited in the preamble that allow to obtain at least one release period of a duration of one hour or less, subsequent to a respective no-release period that largely exceeds 8 hours, e.g. attaining up to 14 hours. There are several possible reasons that make such a long time lag very desirable. The purpose of a long time lag may be to release the active substance in the patient's colon region, or to account for a slowing down of the intestinal transit that may have various causes such as the natural effect of nocturnal rest or the action of some drug or a pathological cause and the like. The purpose of a long time lag may also be to allow a drug to be taken in advance for timing reasons that are external to the patient, for instance in view of circumstances expected to arise within a few hours and that will then make medication required but unpracticable (for instance when travelling, being on stage, taking the floor, taking a school examination or the like).

For the purposes of the present specification, the term "tablet system" shall be construed to mean any kind of drug delivery system adapted for oral delivery to a patient and comprised of a core, a layer provided adjacent to the core to coat and fully enclose the latter, and optionally further successive layers each provided adjacent to a preceding layer to coat and fully enclose the latter.

Accordingly, the present invention provides a tablet system for pharmaceutical use of the kind recited in the preamble, wherein the enclosing element is formulated to have an intrinsic porosity that remains substantially constant upon, and irrespective of, exposure of the enclosing element to the aqueous medium, said porosity allowing and controlling penetration of the aqueous medium, upon said exposure, through the enclosing element towards the enclosed element, said penetration allowing the aqueous medium to eventually reach the enclosed element and then cause an alteration thereof at least at a surface thereof, which alteration causes the enclosing element to experience fragmentation, which fragmentation then allows the aqueous medium to directly reach the enclosed element to provide for rapid establishment and progress to completion of the release of active substance from the enclosed element.

Preferably, the degradation of the enclosed element will cause a swelling or effervescence thereof which causes the enclosing element to become fragmented.

Also preferably, the penetration of the aqueous medium through the enclosing element results in an increase of osmotic pressure within the enclosing element that causes the enclosing element to become fragmented.

Preferably, at least one tablet element of said couple of tablet elements may be formed as a layer and made, using a layer-making procedure selected from film coating and pressing, and also, the tablet elements of said couple of tablet elements both may be formed as layers and made, using different layer-making procedures selected from film coating and pressing, respectively.

Preferably, the enclosing tablet element is selected to provide a corresponding no-release period that is longer than about 8 hours.

In a particularly preferred embodiment, the tablet system is comprised of a plurality of couples of tablet elements and made up of tablet elements adjacent to each other in succession and enclosing each other in succession from an innermost tablet element to an outermost tablet element over the whole plurality of couples, which will allow the tablet system to provide, upon exposure to the aqueous medium, a corresponding plurality of no-release and release periods subsequent to each other in alternate succession.

The present invention also provides a method of making an enclosing element devoid of active substance as defined above in a tablet system as defined above in which the enclosing element is embodied as a film-coated layer, including the step of spray-coating a solution made of components of a film-coating composition dissolved in a pharmaceutically acceptable organic solvent, preferably in ethanol 96 % by volume with the remainder being water.

The tablet system of the invention allows to provide for release of one or more active substances each during a release period subsequent to a no-release period and to produce release and no-release periods independent of each other, for instance any short or long release period independently of the preceding no-release period, whether short or long. More particularly, the active substance that is released last and with a long time lag can yet be released fast, e.g. for optimal drug absorption in the patient's colon region. Specifically, it has been possible to delay the last substance release by a time lag that largely exceeds 8 hours, e.g. attaining up to 14 hours, and yet to provide a duration of this last substance release that does not exceed one hour.

The tablet system of the invention is comprised of layers fully enclosing each other in succession, which construction allows to substantially avoid undesirable fragility that would entail undesired variations of the release rate of the active substance such as "dose dumping" or, on the contrary, release failure due to the coating's collapse.

Also, the simplicity of construction and manufacture of the tablet system of the invention taken together with the free selection of layer-making procedures selected from film coating and pressing allows to produce tablet systems of optimally small size.

More particularly, retarding layers made by film-coating according to the invention have a homogeneous structure that provides a system that is not fragile. This allows to use less material on the tablet and to make the retarding layers thinner, and also allows the spraying process to be faster and easier to control because the film-building material is not diluted with any material that must be added to form channels, to increase the permeability etc., as in prior art tablet systems mentioned above. The inventors' experimental results have shown that the usual variation thickness of retarding layers manufactured industrially (which variation corresponds to about 2 % in increase of the final tablet weight) does not lead to any appreciable variation of the lag time duration. Moreover, the fact that the film-coated retarding layer according to the invention is comprised of less components than usual in prior art film-coated retarding layers reduces the risk of undesirable interaction e.g. with the active substance contained in the underlying element, whether the latter is a layer or the core.

Accordingly, the tablet system of the invention strongly contributes to improve the patient's compliance to the administration of the drugs required by the patient's condition.

It is believed that the results obtained with the tablet system of the invention are due to an initial situation and sequence of events that may be explained as follows, reference being made to an enclosing element (which provides the no-release period) and the underlying enclosed element (which provides the subsequent delayed-release period):

When the enclosing element comes into contact with the aqueous medium, such as gastric or intestinal fluid, depending on the desired location of the release in the patient's body, the enclosing element's intrinsic porosity will allow a controlled penetration of the aqueous medium through the enclosing element and towards the underlying enclosed element. When the aqueous medium eventually reaches the enclosed element, this will cause an alteration of the latter, initially affecting the latter's surface properties, but eventually affecting the latter's body and intrinsic properties, too. The alteration of the enclosed element may be a change of volume due to swelling or effervescence. Alternatively or simultaneously, there will take place an increase of osmotic pressure within the enclosing element. Whatever the case, a force is exerted on the enclosing element that tends to expand the latter, eventually leading to the latter's fragmentation. This fragmentation of the enclosing element then allows the aqueous medium to directly reach the underlying enclosed element, resulting in the desired rapid establishment and progress to completion of the release of active substance from the enclosed element.

This mechanism is believed to explain that a fast release of active substance from the enclosed element can be obtained, whether the preceding no-release duration has been short or long, and more generally, to explain that each release duration obtained by means of the tablet system of the invention is observed to be completely independent of any of the preceding no-release durations.

The observed results also lead to believe that the enclosing element's intrinsic porosity remains substantially constant upon, and irrespective of, exposure of the enclosing element to the aqueous medium.

The observed results also lead to believe that whether or not the enclosing element undergoes a degradation e.g. by erosion, dissolution, digestion, enzymatic biodegradation or even melting (e.g. in the case of a fatty or waxy compound), it is not this degradation mechanism that controls the establishment and progress to completion of the release of active substance from the underlying enclosed element. More particularly, the degradation, if any, of the enclosed element does not appear to increase the latter's intrinsic porosity to an appreciable degree. This latter result is obtained, in the tablet system of the invention, by formulating the enclosing element with compounds that allow to build up a retarding layer that is neither soluble nor permeable to the aqueous medium. More particularly, in making the film-coated retarding layers the use of a pharmaceutically acceptable organic solvent (preferably ethanol 96 % by volume with the remainder being water) allows to control the layers' intrinsic structure and porosity and to obtain retarding layers whose porosity will not be affected appreciably by exposure to the aqueous medium; in making press-coated retarding layers, a proper choice of the retarding layers' components and the parameters of the retarding layers' manufacturing process leads to similar results.

Also, in the tablet system of the invention, the fragmentation of the enclosing element that eventually results from the latter's exposure to the aqueous medium is observed to proceed fast in comparison with any possible outward diffusion of active substance through the pores of the enclosing element when not yet fragmented. Accordingly, in the tablet system of the invention any release of active substance prior to the fragmentation of the enclosing element is negligible, allowing to attain a neat distinction between the no-release period and the subsequent release period.

Such results cannot be attained with systems whose nature and composition will cause the porosity to increase by exposure to the aqueous medium, e.g. with systems according to the above discussed prior art of US-A-5529790.

To provide the desired so-called fast or pulse-like release period, the duration of which desirably does not exceed about one hour, respective elements (core or layer) of the system that are comprised of active substance may be formulated using excipients that promote the element's disintegration. In particular, use may be made of so-called "super-disintegrants" that are known in the art as potent disintegration helpers with the properties i.a.of good water uptake due to high capillary action, rapid swelling leading to a volume increase of up to 500 %, efficient fluid channelling resulting from long fibre length. Such "super-disintegrants" may be comprised of modified starches and cross-linked cellulosic polymers and may be used in concentrations of about 3 % to 5 % by weight, whereas concentrations of about 10 % by weight would be necessary to obtain similar results with other disintegrants. Generally, disintegration promoters can be excipients such as sodium croscarmellose, sodium starch glycolate, modified starch and the like; hydrophilic diluents such as lactose, microcrystalline cellulose, mannitol, starch and starch derivatives; or pharmaceutically acceptable effervescence-causing substances that lead to a fast disintegration of the respective element (core or layer) when the latter is contacted with an aqueous acid medium, more particularly with the gastric medium, such as the carbonates and sodium hydrogen carbonates of sodium and/or other pharmaceutically acceptable alkaline or alkaline earth metals. Pharmaceutically acceptable additives may be incorporated e.g. to promote effervescence, such as citric acid, tartaric acid, fumaric acid and the like, or to raise the osmotic pressure, such as sodium chloride, glucose and the like. Also, excipients well known in the art of tablet system manufacturing may be used, such as polyvinylpyrrolidone, cellulose derivatives (e.g. hydroxypropylcellulose, hydroxypropylmethylcellulose, carboxymethylcellulose and their derivatives) as well as known diluents, lubricants, binders, flow promoters and the like.

To provide the desired no-release period, respective layer elements of the system that are manufactured by film coating procedures may be formulated using substances well known for this purpose in the art of tablet system manufacturing, such as ethylcellulose, cellulose acetate, cellulose acetate butyrate, acrylic and methacrylic polymers and copolymers, polyvinylalcohol, polyvinyl chloride and the like as well as mixtures thereof. Also, excipients well known in the art of tablet system manufacturing may be used, such as dibutylsebacate, glyceryl monostearate and the like, anti-tacking agents (e.g. talc and the like) as well as further excipients well known in the art of film coating in tablet system manufacturing.

To provide the desired no-release period, respective layer elements of the system that are manufactured by pressing procedures may be formulated using substances well known for this purpose in the art of tablet system manufacturing, such as cellulose derivatives as mentioned above, or also fatty acids or their esters, ethers, salts or amines (e.g. stearylamine), long chain aliphatic alcohols, polyoxyethylene alkyl ethers, polyoxyethylene stearates, sugar esters, lauroyl macrogol-32 glyceryl, stearoyl macrogol-32 glyceryl and the like. Also, excipients well known in the art of tablet system manufacturing may be used, such as waxy substances (e.g. carnauba wax, paraffin, microcrystalline wax, beeswax, cetyl ester wax and the like), non-fatty hydrophobic substances such as dibasic calcium phosphate, as well as known diluents, lubricants, binders, flow promoters and the like.

### BRIEF DESCRIPTION OF THE DRAWINGS

- Fig. 1: illustrates a first embodiment of a tablet system according to the present invention schematically shown in a cross-section view;
- Fig. 2: illustrates a second embodiment of a tablet system according to the present invention schematically shown in a cross-section view;
- Fig. 3: illustrates a third embodiment of a tablet system according to the present invention schematically shown in a cross-section view;
- Fig. 4: illustrates a fourth embodiment of a tablet system according to the present invention schematically shown in a cross-section view;
- Fig. 5: is a diagram of a time profile (3 test runs) of active substance release obtained in vitro with a tablet system according to the embodiment of Fig. 1;
- Figs. 6, 7 and 8: are diagrams each of a time profile (3 test runs each) of active substance release obtained in vitro with a tablet system according to the embodiment of Fig. 2;
- Fig. 9: is a time profile diagram of active substance release (3 test runs) obtained in vitro with a tablet system according to the embodiment of Fig. 3;
- Fig. 10: is a time profile diagram of active substance release (3 test runs) obtained in vitro with a tablet system according to the embodiment of Fig. 4;
- Fig. 11: is a picture illustrating the fragmentation caused by dissolution of a tablet system according to the embodiment of Fig. 3.

### DETAILED DESCRIPTION OF THE INVENTION

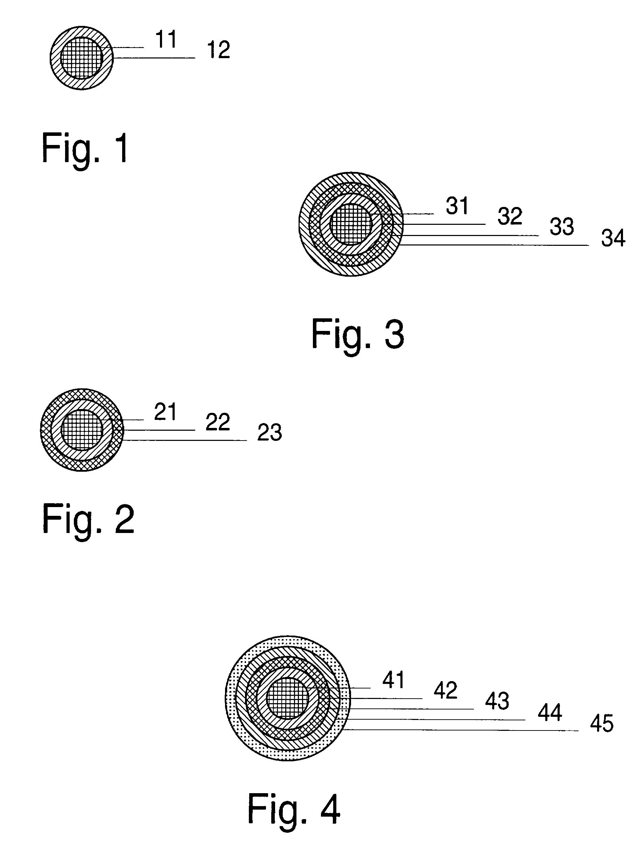

Embodiments of a tablet system according to the present invention are illustrated in Fig. 1 to 4. For the sake of simplification the illustrated tablet systems each time are represented to have a spherical shape and a centred symmetry, which allows the cross-section view through the tablet system schematically shown in Fig. 1 to 4 to suffice to illustrate the respective embodiment. Of course, actual tablet systems may have any shape commonly known and used in the art.

A first - and simplest - embodiment of a tablet system according to the present invention is illustrated in Fig. 1. The tablet system is comprised of two tablet elements that are, enumerated in succession from the centre outwards, a core 11 and an external coating layer 12 that completely encloses the core 11 and is adjacent thereto, thus providing a complete coating of the tablet system. It will be appreciated that in this manner the tablet system illustrated in Fig. 1 is made up of a core-centred body comprised of one couple of tablet elements adjacent to each other, one of which (the core 11) is fully enclosed within the other one (the external coating layer 12), which defines a couple of tablet elements that are associated with each other as enclosed and enclosing tablet elements, respectively.

This tablet system is for pharmaceutical use i.e. intended to deliver an active substance to the body of a patient who takes, e.g. swallows, the tablet system. To this effect, the core 11 is formulated to contain the active substance that can be released in the course of a respective release period in a manner that will be described more in detail below. The external coating layer 12 is formulated devoid of active substance. Thus, in the course of the degradation of the tablet system in the patient's body the external coating layer 12 will provide a no-release period that precedes the release period of the active substance.

In the context of this embodiment as well as all other embodiments described below:
(a) the term "active substance" shall be construed herein to include any single compound or mixture of compounds contained in the enclosed tablet element, excluding any effective compound or mixture that may be contained in the enclosing tablet element; and
(b) the term "no-release" shall be construed herein to refer only to the above mentioned active substance i.e. it is only the above mentioned active substance that shall not be released during the no-release period.

Thus, in the embodiment of a tablet system illustrated in Fig. 1, if desired, the external coating layer 12 may be formulated to contain some other effective compound or mixture of compounds that is not the active substance defined above under (a) and will eventually be released in the course of the no-release period defined above, irrespective and independent of what is described herein with regard to the active substance defined above under (a).

To provide a predetermined duration of the no-release period, the external coating layer 12 is formulated to have an intrinsic porosity that remains substantially constant when the tablet system comes into contact with an aqueous medium such as saliva, gastric or intestinal fluid, depending on the desired location of the release in the patient's body. This porosity allows and controls the penetration of the aqueous medium through the external coating layer 12 towards the core 11.

The core 11 is formulated with excipients that experience a degradation when the aqueous medium reaches it. Hence, the aqueous medium will cause an alteration at least of the surface and possibly even of the volume of the core 11. To provide one variant of the alteration mechanism, excipients may be selected that will experience swelling when contacted with the aqueous medium, which will cause a swelling of the core 11. To provide another variant of the alteration mechanism, excipients may be selected that will experience effervescence when contacted with the aqueous medium, which will cause an effervescence of the core 11. To provide still another variant of the alteration mechanism, excipients may be selected whose contact with the aqueous medium will cause an increase of osmotic pressure within the external coating layer 12 that encloses the core 11. In each of these variants of the alteration mechanism, the resulting alteration of the core 11 will cause the external coating layer 12 to become fragmented, which in turn will allow the aqueous medium to reach the core 11 more freely and possibly the fragmentation of the external coating layer 12 to self-accelerate. Eventually, the core 11 will release the active substance while undergoing essentially a complete degradation that will proceed during a release period, the predetermined duration of which corresponds to the time needed for the core 11 to undergo an essentially complete degradation in the patient's body.

In the succession of events described above, there can be discerned an initial stage at which the aqueous medium penetrates into the external coating layer 12 but does not yet reach the core 11, an intermediate stage at which the porosity of the external coating layer 12 allows the aqueous medium to reach the core 11 more and more, and a final stage at which the external coating layer 12 has become fragmented to such an extent that the aqueous medium can be considered to reach the core 11 freely. A proper selection of excipients allows to make the duration of the intermediate stage i.e. the time interval between the initial and final stages independent of the duration of the initial and final stages, hence making it possible to select the excipients used in the core 11 and external coating layer 12, respectively, to keep the duration of the intermediate stage short in comparison with the duration of the initial and final stages, more particularly in comparison with the final stage, which is the duration of the release period.

In this context, in analogy with the above defined "fast" release where 80 % of the release takes place within one hour or less, the term "short" applied to time may be construed herein to mean that of two durations the ratio of the shorter one to the longer one will be less than about 20:80.

A second embodiment of a tablet system according to the present invention is illustrated in Fig. 2. This tablet system is comprised of three tablet elements that are, enumerated in succession from the centre outwards, a core 21 comprised of active substance, a single intermediate layer 22 devoid of active substance and an external coating layer 23 comprised of active substance. The intermediate layer 22 completely encloses the core 21 and is adjacent thereto. The external coating layer 23 completely encloses the intermediate layer 22 and is adjacent thereto, thus providing a complete coating of the tablet system. It will be appreciated that in this manner the tablet system illustrated in Fig. 2 is made up of a core-centred body, and that two of the three tablet elements that are adjacent to each other form a couple of the kind defined herein, the tablet elements of which are adjacent to each other and associated with each other as enclosed and enclosing, namely, the core 21 that is comprised of active substance and enclosed within the intermediate layer 22 that is devoid of active substance.

In analogy with the tablet system described above and illustrated in Fig. 1, the tablet system illustrated in Fig. 2 is for pharmaceutical use i.e. intended to deliver one or more active substances to the body of a patient who takes the tablet system. To this effect, the core 21 is formulated to contain a first active substance that can be released in the course of a respective release period. The external coating layer 23 is formulated to contain a second active substance that can be released in the course of a respective release period. The intermediate layer 22 is formulated devoid of both the first and second active substances. Thus, in the course of the degradation of the tablet system in the patient's body the intermediate layer 22 will provide a no-release period that separates the respective release periods pertaining to the first and second active substances. Incidentally, the intermediate layer 22 need not be devoid of any effective substance of interest i.e. if desired it may contain some effective substance different from the first and second active substances.

As the first and second active substances may either be the same or different ones, it will be appreciated that in a first variant the tablet system of this embodiment can be made to release a single active substance in the course of two release periods that are separated from each other by the no-release period, whereas in a second variant the tablet system can be made to initially release the first active substance and then, after the no-release period, to release the second active substance.

For the sake of clarity it is noted that the first active substance is defined to be contained in the core 21 and thus, in the course of the degradation of the tablet system in the patient's body it will be released last, whereas the second active substance is defined to be contained in the external coating layer 23 and will thus be released earliest. Accordingly, the successive release of the active substances will appear to occur in reverted order with regard to the respective designation of the substances as first and second as used herein.

To provide a predetermined duration of the earliest-occurring release of active substance, i.e the release of the second active substance, the external coating layer 23 is formulated to contain the second active substance vehiculated with excipients resulting in a controlled degradation rate of the external coating layer 23 and concomitant release of the second active substance when the tablet system comes into contact with an aqueous medium such as saliva, gastric or intestinal fluid, depending on the desired location of the release in the patient's body. The release of the second active substance will take place during a respective release period, the predetermined duration of which corresponds to the time needed for the external coating layer 23 to undergo an essentially complete degradation in the patient's body.

In similar manner, to provide a predetermined duration of the last-occurring release of active substance, i.e the release of the first active substance, the core 21 is formulated to contain the first active substance vehiculated with excipients resulting in a controlled degradation rate of the core 21 and concomitant release of the first active substance during a respective release period, the predetermined duration of which corresponds to the time needed for the core 21 to undergo an essentially complete degradation in the patient's body.

In analogy with the preceding and also the description given above of the tablet system illustrated in Fig. 1, to provide a predetermined duration of the no-release period that separates the respective release periods of the first and second active substances the intermediate layer 22 is formulated to have an intrinsic porosity that remains substantially constant when the tablet system comes into contact with the aqueous medium and allows and controls the penetration of the aqueous medium through the intermediate coating layer 22 towards the core 21. The resulting alteration of the core 21 will cause the intermediate coating layer 22 to become fragmented, which in turn will allow the aqueous medium to reach the core 21 more freely and possibly the fragmentation of the intermediate coating layer 22 to self-accelerate. Eventually, the core 21 will release the active substance, as has been described above in respect of the tablet system illustrated in Fig. 1.

In close analogy with the first and second embodiments of a tablet system described above and illustrated in Figs. 1 and 2, a third embodiment of a tablet system according to the present invention is illustrated in Fig. 3. This tablet system is comprised of four tablet elements that are, enumerated in succession from the centre outwards, a core 31 comprised of active substance, an innermost intermediate layer 32 devoid of active substance, an outermost intermediate layer 33 comprised of active substance and an external coating layer 34 devoid of active substance. The innermost intermediate layer 32 completely encloses the core 31 and is adjacent thereto. The outermost intermediate layer 33 completely encloses the innermost intermediate layer 32 and is adjacent thereto. The external coating layer 34 completely encloses the outermost intermediate layer 33 and is adjacent thereto, thus providing a complete coating of the tablet system. It will be appreciated that in this manner the tablet system illustrated in Fig. 3 is made up of a core-centred body and comprised of two couples of the kind defined herein, the tablet elements of which are adjacent to each other and associated with each other as enclosed and enclosing, namely, a first couple is made up of the core 31 that is comprised of active substance and enclosed within the innermost intermediate layer 32 that is devoid of active substance, and a second couple is made up of the outermost intermediate layer 33 that is comprised of active substance and enclosed within the external coating layer 34 that is devoid of active substance.

Still in close analogy with the first and second embodiments of a tablet system described above and illustrated in Figs. 1 and 2, this third embodiment of a tablet system according to the present invention is for pharmaceutical use. The core 31 is formulated to contain a first active substance that can be released in the course of a respective release period. The innermost intermediate layer 32 is formulated devoid of the first active substance to provide a no-release period that immediately precedes the release period associated with the first active substance and thus, separates the respective release periods pertaining to the first and second active substances. The outermost intermediate layer 33 is formulated to contain a second active substance that can be released in the course of a respective release period. The external coating layer 34 is formulated devoid of the second active substance to provide a no-release period that immediately precedes the release period associated with the second active substance. The first and second active substances may either be the same or different ones. Either or both the innermost intermediate layer 32 and the external coating layer 34 need not be devoid of any effective substance i.e. if desired either or both may contain some effective substance different from the first and second active substances.

In close analogy with the first, second and third embodiments of a tablet system described above and illustrated in Figs. 1, 2 and 3, a fourth embodiment of a tablet system according to the present invention is illustrated in Fig. 4 that is comprised of five tablet elements that are, enumerated in succession from the centre outwards, a core 41 comprised of active substance, an innermost intermediate layer 42 devoid of active substance, an auxiliary intermediate layer 43 comprised of active substance, an outermost intermediate layer 44 devoid of active substance and an external coating layer 45 comprised of active substance, so that the tablet system illustrated in Fig. 4 is made up of a core-centred body comprised of two couples of the kind defined herein, the tablet elements of which are adjacent to each other and associated with each other as enclosed and enclosing, namely, a first couple is made up of the core 41 that is comprised of active substance and enclosed within the innermost intermediate layer 42 that is devoid of active substance, and a second couple is made up of the outermost intermediate layer 43 that is comprised of active substance and enclosed within the external coating layer 44 that is devoid of active substance.

It will be appreciated by persons skilled in the art that further embodiments can easily be devised that are made up of a core-centred body comprised of five or more couples of tablet elements that are adjacent to each other and associated with each other as enclosed and enclosing, respectively.

In all embodiments of a tablet system according to the present invention described above, any of the intermediate layer(s) and external coating layer may be made, using layer-making procedures well known in the art of making layers on tablet systems for pharmaceutical use, more particularly by film coating and pressing procedures that may be the same or different ones for each of the layers, as desired.

Also, in the formulation of the core, the intermediate layer(s) and the external coating layer, suitable pharmaceutically acceptable compounds are used together with the active substance, if any, to provide the desired mechanism and rate of degradation of the core or layer. Such compounds may be, typically and by way of example, selected from one or more of the substances known in the art and disclosed e.g. in EP-A-0788790 in their respective "drug delivery", "barrier" and "effervescent" functions. Adjuvant substances normally employed in the pharmaceutical technique may also be used and added, such as diluents, buffers, binders, absorbents, lubricants etc. also as disclosed e.g. in EP-A-0788790.

It will be appreciated that with the exception the first embodiment illustrated in Fig. 1, the core of a tablet system according to the present invention may as well be formulated devoid of an active substance as defined above. Indeed, in a multiple-pulse drug delivery system each and any of the tablet elements may be formulated to contain some other effective substance that is not the one active substance, or any of the active substances, delivered in multiple-pulse releases.

As will be shown in the Examples given below, the invention allows to make tablet systems that provide a no-release period of about 8 hours and more, which has not been obtainable hitherto in comparable systems.

### EXAMPLE 1

### Preparation of diltiazem HCl two-element tablet systems with a pressed core and a spray-coated external coatinq layer

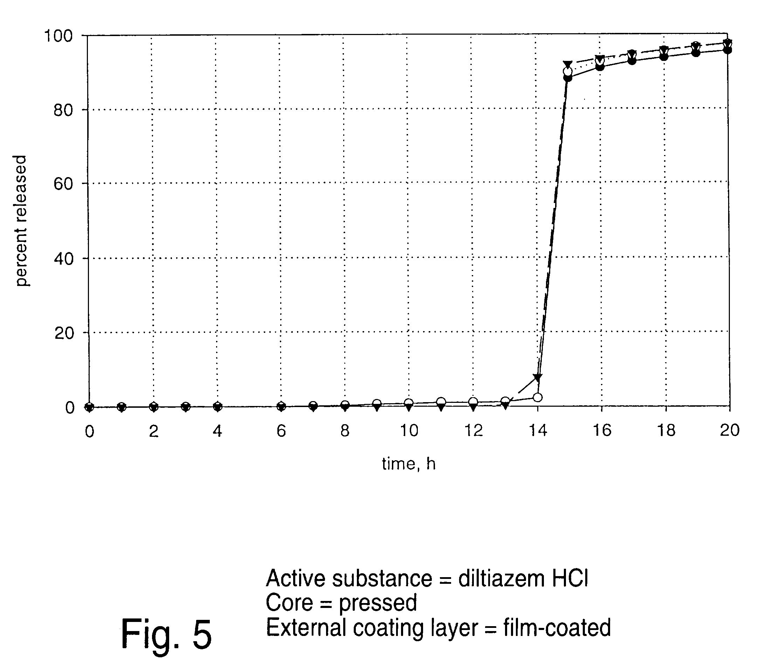

Tablets of the type illustrated in Fig. 1 were prepared, comprised of a core enclosed within an external coating layer. These elements were produced by means of pressing the core and spray-coating thereon a film of external coating layer. Resulting release characteristics of these tablet systems are illustrated in Fig. 5 that will be described below.

### 1.a Preparation of a qranulate adapted to form the core

The required amount of granulate was prepared to allow the production of 2,000 effervescent cores each having a weight of about 468 mg and the following composition:

| | |
|---|---|
| diltiazem HCl | 80.00 mg |
| citric acid | 110.40 mg |
| (Merck, Germany) | |
| sodium hydrogen carbonate | 49.60 mg |
| (CFS, Switzerland) | |
| microcrystalline cellulose | 80.00 mg |
| (Avicel PH102^{(R)}, FMC Corporation, USA) | |
| lactose (lactose pulvis H20) | 120.00 mg |
| (Lactose Fast Flo^{(R)}, Foremost, USA) | |
| sodium croscarmellose | 20.00 mg |
| (Ac-Di-Sol^{(R)}, FMC Corporation, USA) | |
| magnesium stearate | 7.00 mg |
| (Merck, Germany) | |
| colloidal silica | 1.00 mg |
| (Aerosil^{(R)} 200, Degussa AG, Hanau, Germany) | |

An effervescent granulate was prepared by mixing the proper amounts of citric acid and sodium hydrogen carbonate in a high shear mixer type Niro-Fielder PP1 (Aeromatic-Fielder AG, Switzerland) so as to form a mixture of substances capable of effervescence. The homogeneous mixture was then wetted with ethanol (96 % by volume, remainder water). After massing, the granulate so obtained was dried in a fluidised air bed drier type Niro-Aeromatic Strea I (60°C inlet air temperature) (Aeromatic-Fielder AG, Switzerland) and then calibrated on a 0.8 mm aperture sieve granulator type Frewitt GLA (Frewitt Fabriques de Machines SA, Switzerland). The granulate was placed in a cubic mixer type Erweka (Mapag Machine et Plastique SA, Switzerland), added with proper amounts of diltiazem HCl, sodium croscarmellose, microcrystalline cellulose, lactose and colloidal silica, and mixed for 15 min at 22 osc/min. Then, a proper amount of magnesium stearate was added, and mixing was continued for 5 min. This mixture was then used in the compression procedure described below.

### 1.b Preparation of a composition adapted to form the external coating layer

The required amount of a film-forming composition was prepared to allow the production of 2,000 external coating layers each making up 10.5 percent by weight of the tablet system after completed spraying. The film-forming composition contained the following:

| | |
|---|---|
| ethylcellulose | g90.00 |
| (Aqualon^{(R)} N10, Aqualon, France) | |
| dibutyl sebacate | 9.0 g |
| (Aceto Corporation, USA) | |
| ethanol (96 % by vol., remainder water) | 901.00 g |

A homogeneous composition was prepared by placing the proper amounts of dibutyl sebacate and ethanol in a beaker and subjecting the mixture to magnetic stirring, then adding the proper amount of ethylcellulose and continuing the stirring. This mixture was then used in the spraying procedure described below.

### 1.c Preparation of the cores

The cores were prepared by means of a rotating press machine type Kilian RUD (Kilian & Co. GmbH, Germany) equipped with circular convex punches having a 10 mm diameter, operating on the granulate prepared as described above under 1.a.

### 1.d Application of the external coating layers onto the cores

The cores previously prepared as described above under 1.c each were spray-coated with a film of external coating layer by means of a pan coater type Glatt GC250 (Maschinen und Apparatebau AG, Switzerland), operating on the film-forming composition prepared as described above under 1.b. The suspension of film-forming composition was maintained under magnetic stirring at all times during this procedure and sprayed onto the cores from step 1.c at a rate of 20 g/min while the tablet system bed temperature was maintained at 30°C. The spray-coated tablet systems were dried in the pan coater for 10 min while the temperature was maintained at 30°C.

### 1.e Release characteristics of the tablet systems (Fig. 5)

To determine the release characteristics of the tablet systems prepared as described above under 1.a to 1.d a standard equipment was used that is defined in the United States Pharmacopoeia (USP) XXIII, Chapter 711, page 1792, paragraph "Apparatus 2". This equipment has a stirring paddle formed from a blade and a shaft and was operated at 100 rpm. An aqueous solution of 60 mmol/l TRIS (i.e. trometamol) and 90 mmol/l NaCl at 37°C was used as dissolution medium. The release of active substance (diltiazem HCl) was monitored by UV spectrophotometry at 240 nm. With this equipment the *in vitro* release of the active substance from the tablet systems was investigated for 3 sample tablet systems subjected to respective dissolution runs. The investigation results are illustrated in Fig. 5 as respective time profile diagrams. The characteristics that appear therefrom will be discussed below.

### INTERPRETATION OF THE TIME PROFILE DIAGRAM OF EXAMPLE 1

From the time profile diagram of Example 1 illustrated in Fig. 5 the following characteristics clearly appear:

At first a no-release period can be observed that is due to the external coating layer, followed by a release period in which the active substance originates from the core. The no-release period appears as a well-defined time interval having a duration of about 13 to 14 hours, observed between the start of the experiment and the start of the release. The release of active substance is observed to take place within a release period of less than one hour duration.

### EXAMPLE 2

### Preparation of verapamil HCI three-element tablet systems with a pressed core and press-coated intermediate and external coating layers

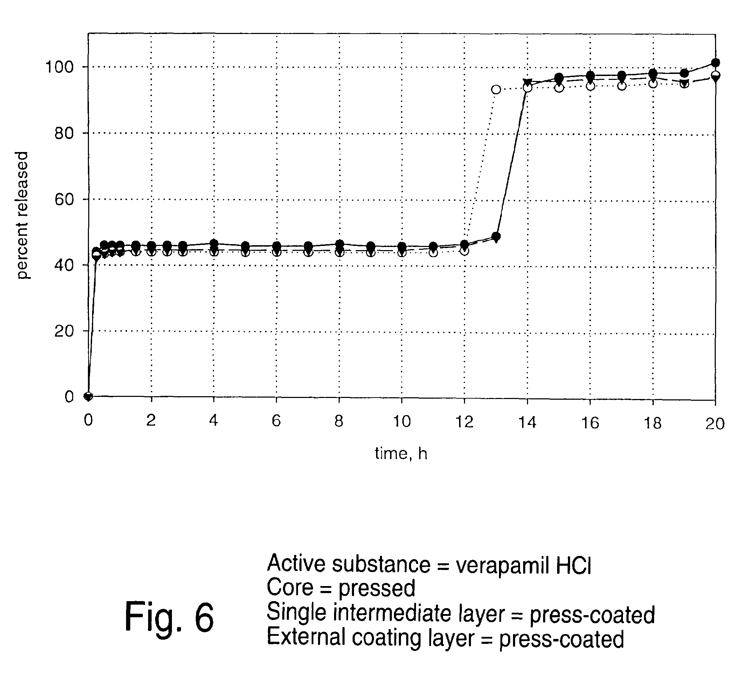

Tablets of the type illustrated in Fig. 2 were prepared, comprised of a core enclosed within an intermediate layer itself enclosed within an external coating layer. The elements were produced by means of pressing the core and press-coating the layers thereon in succession. Resulting release characteristics for these tablet systems are illustrated in Fig. 6 that will be described below.

### 2.a Preparation of a granulate adapted to form the core

The required amount of granulate was prepared to allow the production of 3,000 effervescent cores each having a weight of about 170 mg and the following composition:

| | |
|---|---|
| verapamil HCl | 60.00 mg |
| citric acid | 34.90 mg |
| (Merck, Germany) | |
| sodium hydrogen carbonate | 15.70 mg |
| (CFS, Switzerland) | |
| microcrystalline cellulose | 47.00 mg |
| (Avicel PH102^{(R)}, FMC Corporation, USA) | |
| sodium croscarmellose | 10.00 mg |
| (Ac-Di-Sol^{(R)}, FMC Corporation, USA) | |
| magnesium stearate | 1.70 mg |
| (Merck, Germany) | |
| colloidal silica | 0.70 mg |
| (Aerosil^{(R)} 200, Degussa AG, Hanau, Germany) | |

An effervescent granulate was prepared by mixing the proper amounts of citric acid and sodium hydrogen carbonate in a high shear mixer type Niro-Fielder PP1 (Aeromatic-Fielder AG, Switzerland) so as to form a mixture of substances capable of effervescence. The homogeneous mixture was then wetted with ethanol (96 % by volume, remainder water). After massing, the granulate so obtained was dried in a fluidised air bed drier type Niro-Aeromatic Strea I (60°C inlet air temperature) (Aeromatic-Fielder AG, Switzerland) and then calibrated on a 0.8 mm aperture sieve granulator type Frewitt GLA (Frewitt Fabriques de Machines SA, Switzerland). The granulate was placed in a cubic mixer type Erweka (Mapag Machine et Plastique SA, Switzerland), added with proper amounts of verapamil HCl, sodium croscarmellose, microcrystalline cellulose and colloidal silica, and mixed for 15 min at 22 osc/min. Then, a proper amount of magnesium stearate was added, and mixing was continued for 5 min. This mixture was then used in the compression procedure described below.

### 2.b Preparation of a granulate adapted to form the intermediate layer

The required amount of granulate was prepared to allow the production of 3,000 intermediate layers each having a weight of about 600 mg and the following composition:

| | |
|---|---|
| dibasic calcium phosphate | 300.00 mg |
| (Emcompress, Mendell, USA) | |
| glyceryl behenate | 240.00 mg |
| (Compritol^{(R)} 888 ATO, Gattefossé, France) | |
| polyvinylpyrrolidone | 51.00 mg |
| (Plasdone^{(R)} K29-32, ISP AG, Switzerland) | |
| magnesium stearate | 6.00 mg |
| (Merck, Germany) | |
| colloidal silica | 3.00 mg |
| (Aerosil^{(R)} 200, Degussa AG, Hanau, Germany) | |

A granulate was prepared by mixing the proper amounts of dibasic calcium phosphate, glyceryl behenate and polyvinylpyrrolidone in a high shear mixer type Niro-Fielder PP1 (Aeromatic-Fielder AG, Switzerland). The homogeneous mixture was then wetted with demineralised water. After massing, the granulate so obtained was dried in a fluidised air bed drier type Niro-Aeromatic Strea I (60°C inlet air temperature) (Aeromatic-Fielder AG, Switzerland) and then calibrated on a 0.8 mm aperture sieve granulator type Frewitt GLA (Frewitt Fabriques de Machines SA, Switzerland). The granulate was placed in a cubic mixer type Erweka (Mapag Machine et Plastique SA, Switzerland), added with a proper amount of colloidal silica, and mixed for 15 min at 22 osc/min. Then, a proper amount of magnesium stearate was added, and mixing was continued for 5 min. This mixture was then used in the compression procedure described below.

### 2.c Preparation of a granulate adapted to form the external coating layer

The required amount of granulate was prepared to allow the production of 3,000 external coating layers each having a weight of about 350 mg and the following composition:

| | |
|---|---|
| verapamil HCl | 60.00 mg |
| microcrystalline cellulose | 110.00 mg |
| (Avicel PH102^{(R)}, FMC Corporation, USA) | |
| lactose (lactose pulvis H20) | 130.00 mg |
| (Lactose Fast Flo^{(R)}, Foremost, USA) | |
| sodium croscarmellose | 45.50 mg |
| (Ac-Di-Sol^{(R)}, FMC Corporation, USA) | |
| magnesium stearate | 3.00 mg |
| (Merck, Germany) | |
| colloidal silica | 1.50 mg |
| (Aerosil^{(R)} 200, Degussa AG, Hanau, Germany) | |

A granulate was prepared by placing the proper amounts of verapamil HCl, microcrystalline cellulose, lactose, sodium croscarmellose and colloidal silica in a cubic mixer type Erweka (Mapag Machine et Plastique SA, Switzerland), and mixing for 15 min at 22 osc/min. Then, a proper amount of magnesium stearate was added, and mixing was continued for 5 min. This mixture was then used in the compression procedure described below.

### 2.d Preparation of the cores

The cores were prepared by means of a rotating press machine type Kilian RUD (Kilian & Co. GmbH, Germany) equipped with circular convex punches having a 7 mm diameter, operating on the granulate prepared as described above under 2.a.

### 2.e Application of the intermediate layers onto the cores

The cores previously prepared as described above under 2.d each were press-coated with an intermediate layer by means of a rotating press machine type Kilian RUD (Kilian & Co. GmbH, Germany) equipped with dies and circular convex punches having a 10 mm diameter, operating on the granulate prepared as described above under 2.b. First and second loading hoppers are filled up with the granulate. Between the two loading hoppers the machine is equipped with a transfer system adapted to feed the cores and exactly position them in the die. For each core, the first loading hopper supplies 300 mg of granulate, which is half the quantity of granulate finally to be applied to the core. Then, a feeding system provides and positions a core exactly centred in the die. Subsequently, the second loading hopper also supplies 300 mg of granulate, which is the other half of the quantity of granulate to be applied to the core. The compression step was then performed.

### 2.f Application of the external coating layers onto the intermediate layers

The half-finished tablet systems made up of cores previously press-coated with an intermediate layer as described above under 2.e each were press-coated with an external coating layer by means of a rotating press machine type Kilian RUD (Kilian & Co. GmbH, Germany) equipped with dies and circular convex punches having a 12 mm diameter, operating on the granulate prepared as described above under 2.c. First and second loading hoppers are filled up with the granulate. Between the two loading hoppers the machine is equipped with a transfer system adapted to feed the coated cores from step 2.e and exactly position them in the die. For each coated core from step 2.e, the first loading hopper supplies 175 mg of granulate, which is half the quantity of granulate finally to be applied to the core. Then, a feeding system provides and positions a coated core from step 2.e exactly centred in the die. Subsequently, the second loading hopper also supplies 175 mg of granulate, which is the other half of the quantity of granulate to be applied to the coated core from step 2.e. The compression step was then performed.

### 2.g Release characteristics of the tablet systems (Fig. 6)

To determine the release characteristics of the tablet systems prepared as described above under 2.a to 2.f a standard equipment was used that is defined in the United States Pharmacopoeia (USP) XXIII, Chapter 711, page 1792, paragraph "Apparatus 2". This equipment has a stirring paddle formed from a blade and a shaft and was operated at 100 rpm. Intestinal fluid at 37°C was used as dissolution medium. The release of active substance (verapamil HCl) was monitored by UV spectrophotometry at 278 nm. With this equipment the *in vitro* release of the active substance from the tablet systems was investigated for 3 sample tablet systems subjected to respective dissolution runs. The investigation results are illustrated in Fig. 6 as respective time profile diagrams. The characteristics that appear therefrom will be discussed below.

### EXAMPLE 3

### Preparation of verapamil HCl three-element tablet systems with a pressed core, a press-coated intermediate layer and a spray-coated external coating layer

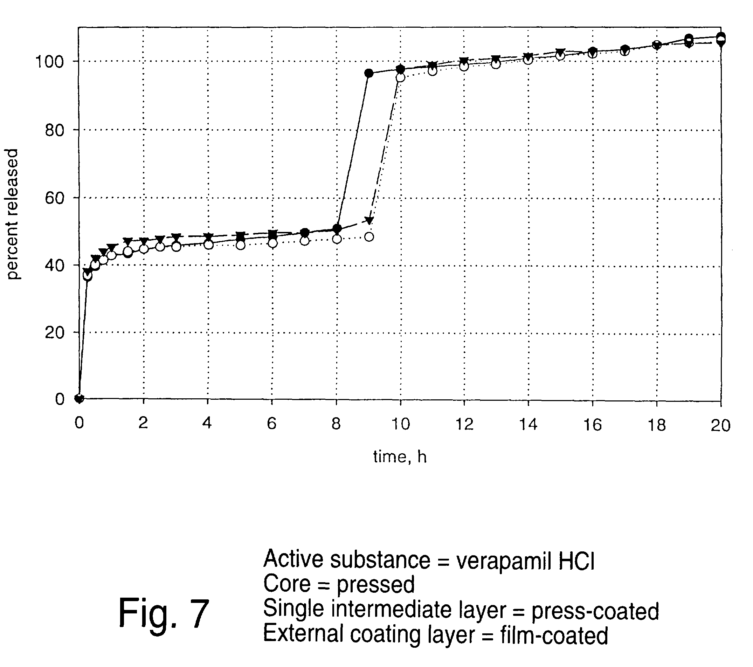

Tablets of the type illustrated in Fig. 2 were prepared, comprised of a core enclosed within an intermediate layer itself enclosed within an external coating layer. The elements were produced by means of pressing the core, press-coating the intermediate layer thereon, and spray-coating onto the intermediate layer a film of external coating layer. Resulting release characteristics of these tablet systems are illustrated in Fig. 7 that will be described below.

### 3.a Preparation of a granulate adapted to form the core

The required amount of granulate had a composition and was prepared as described above in Example 2 under 2.a.

### 3.b Preparation of a granulate adapted to form the intermediate layer

The required amount of granulate had a composition and was prepared as described above in Example 2 under 2.b.

### 3.c Preparation of a composition adapted to form the external coating layer

The required amount of a film-forming composition was prepared to allow the production of 1,300 external coating layers each containing 60 mg of active substance. The film-forming composition contained the following:

| | |
|---|---|
| verapamil HCl | 77.90 g |
| Opadry^{(R)} OY-L | 50.00 g |
| (Colorcon, England) | |
| demineralised water | 366.00 g |

A homogeneous composition was prepared by placing the proper amounts of verapamil HCl, Opadry^{(R)} OY-L and demineralised water in a beaker and subjecting the mixture to magnetic stirring. This mixture was then used in the spraying procedure described below.

### 3.d Preparation of the cores

The cores were prepared as described above in Example 2 under 2.d.

### 3.e Application of the intermediate layers onto the cores

The cores previously prepared as described above under 3.d each were press-coated with an intermediate layer as described above in Example 2 under 2.e.

### 3.f Application of the external coating layers onto the intermediate layers

The half-finished tablet systems made up of cores previously press-coated with an intermediate layer as described above under 2.e each were spray-coated with a film of external coating layer by means of a pan coater type Glatt GC250 (Maschinen und Apparatebau AG, Switzerland), operating on the film-forming composition prepared as described above under 3.c. The suspension of film-forming composition was maintained under magnetic stirring at all times during this procedure and sprayed onto the coated cores from step 3.e at a rate of 10 g/min while the tablet system bed temperature was maintained at 40°C. The spray-coated tablet systems were dried in the pan coater for 30 min while the temperature was maintained at 40°C.

### 3.g Release characteristics of the tablet systems (Fig. 7)

The release characteristics of the tablet systems prepared as described above under 3.a to 3.f were determined as described above in Example 2 under 2.g. The *in vitro* release of the active substance from the tablet systems was investigated for 3 sample tablet systems subjected to respective dissolution runs. The investigation results are illustrated in Fig. 7 as respective time profile diagrams. The characteristics that appear therefrom will be discussed below.

### EXAMPLE 4

### Preparation of bucindolol HCl three-element tablet systems with a pressed core and spray-coated intermediate and external coating layers

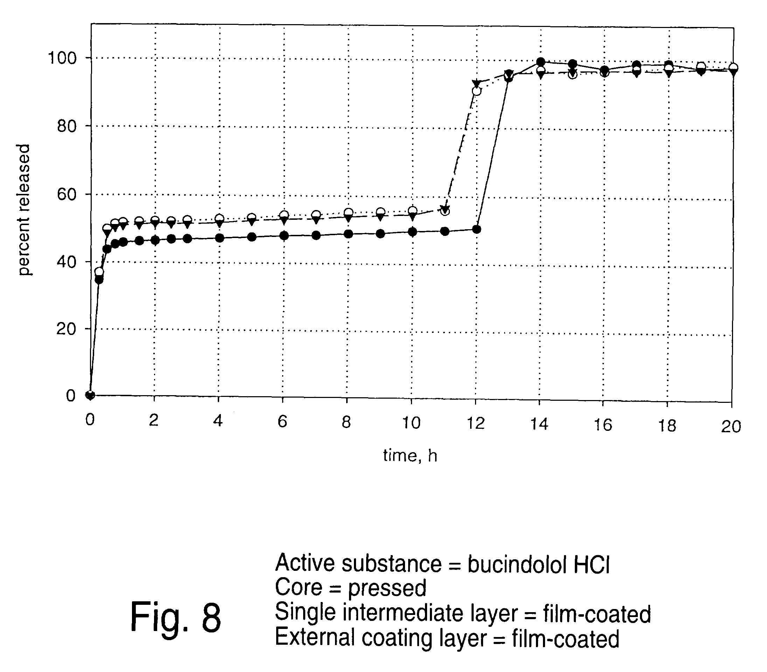

Tablets of the type illustrated in Fig. 2 were prepared, comprised of a core enclosed within an intermediate layer itself enclosed within an external coating layer. The elements were produced by means of pressing the core, spray-coating thereon a film of intermediate coating layer and spray-coating on the latter a film of external coating layer. Resulting release characteristics for these tablet systems are illustrated in Fig. 8 that will be described below.

### 4.a Preparation of a granulate adapted to form the core

The required amount of granulate was prepared to allow the production of 2,700 effervescent cores each having a weight of about 369.25 mg and the following composition:

| | |
|---|---|
| bucindolol HCl | 50.00 mg |
| citric acid | 89.70 mg |
| (Merck, Germany) | |
| sodium hydrogen carbonate | 40.30 mg |
| (CFS, Switzerland) | |
| poly(ethylene glycol) | 4.00 mg |
| (Lutrol^{(R)} E6000, BASF, Switzerland) | |
| microcrystalline cellulose | 90.00 mg |
| (Avicel PH102^{(R)}, FMC Corporation, USA) | |
| lactose (lactose pulvis H20) | 78.65 mg |
| (Lactose Fast Flo^{(R)}, Foremost, USA) | |
| sodium croscarmellose | 15.00 mg |
| (Ac-Di-Sol^{(R)}, FMC Corporation, USA) | |
| magnesium stearate | 1.60 mg |
| (Merck, Germany) | |
| colloidal silica | 0.75 mg |
| (Aerosil^{(R)} 200, Degussa AG, Hanau, Germany) | |

An effervescent granulate was prepared by means of a procedure similar to that described above in Example 2 under 2.a but for using the composition recited above. This mixture was then used in the compression procedure described below.

### 4.b Preparation of a composition adapted to form the intermediate layer

The required amount of a film-forming composition was prepared to allow the production of 2,700 intermediate layers each making up 21 percent by weight of the tablet system after completed spraying. The film-forming composition contained the following:

| | |
|---|---|
| ethylcellulose | 200.00 g |
| (Aqua1on^{(R)} N10, Aqualon, France) | |
| dibutyl sebacate | 10.00 g |
| (Aceto Corporation, USA) | |
| ethanol (96 % by vol., remainder water) | 1780.00 g |

A homogeneous composition was prepared by placing the proper amounts of dibutyl sebacate and ethanol in a beaker and subjecting the mixture to magnetic stirring, then adding the proper amount of ethylcellulose and continuing the stirring. This mixture was then used in the spraying procedure described below.

### 4.c Preparation of a composition adapted to form the external coating layer

The required amount of a film-forming composition was prepared to allow the production of 2,200 external coating layers each containing 50 mg of active substance. The film-forming composition contained the following:

| | |
|---|---|
| bucindolol HCl | 110.00 g |
| Opadry^{(R)} OY-L | 40.00 g |
| (Colorcon, England) | |
| demineralised water | 549.50 g |

A homogeneous composition was prepared by placing the proper amounts of bucindolol HCl, Opadry^{(R)} OY-L and demineralised water in a beaker and subjecting the mixture to magnetic stirring. This mixture was then used in the spraying procedure described below.

### 4.d Preparation of the cores

The cores were prepared as described above in Example 2 under 2.d but for using the composition recited above under 4.a instead of that recited in Example 2 under 2.a.

### 4.e Application of the intermediate layers onto the cores

The cores previously prepared as described above under 4.d each were spray-coated with a film of intermediate layer by means of a pan coater type Glatt GC250 (Maschinen und Apparatebau AG, Switzerland), operating on the film-forming composition prepared as described above under 4.b. The suspension of film-forming composition was maintained under magnetic stirring at all times during this procedure and sprayed onto the cores from step 4.d at a rate of 20 g/min while the tablet system bed temperature was maintained at 30°C. The spray-coated tablet systems were dried in the pan coater for 10 min while the temperature was maintained at 30°C.

### 4.f Application of the external coating layers onto the intermediate layers

The half-finished tablet systems made up of cores previously spray-coated with a film of intermediate layer as described above under 4.e each were spray-coated with a film of external coating layer as described above in Example 3 under 3.f but for using the composition recited above under 4.c instead of that recited in Example 3 under 3.c.

### 4.g Release characteristics of the tablet systems (Fig. 8)

The release characteristics of the tablet systems prepared as described above under 4.a to 4.f were determined as described above in Example 2 under 2.g, except that 50 mmol/l acetate buffer was used as dissolution medium instead of the intestinal fluid at 37°C and the release of active substance (bucindolol HCl) was monitored by UV spectrophotometry at 247 nm instead of 278 nm. The *in vitro* release of the active substance from the tablet systems was investigated for 3 sample tablet systems subjected to respective dissolution runs. The investigation results are illustrated in Fig. 8 as respective time profile diagrams. The characteristics that appear therefrom will be discussed below.

### EXAMPLE 5

### Preparation of diltiazem HCl four-element tablet systems with a pressed core and press-coated layers

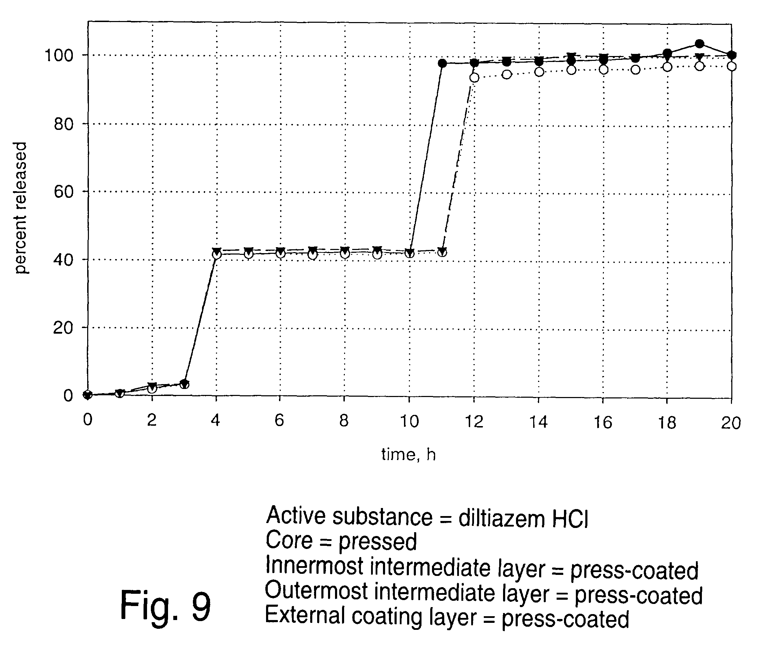

Tablets of the type illustrated in Fig. 3 were prepared, comprised of a core enclosed within an innermost intermediate layer itself enclosed within an outermost intermediate layer itself enclosed within an external coating layer. The elements were produced by means of pressing the core and press-coating the layers thereon in succession. Resulting release characteristics for these tablet systems are illustrated in Fig. 9 that will be described below.

### 5.a Preparation of a granulate adapted to form the core

The required amount of granulate was prepared to allow the production of 2,000 effervescent cores each having a weight of about 150.18 mg and the following composition:

| | |
|---|---|
| diltiazem HCl | 17.00 mg |
| citric acid | 66.00 mg |
| (Merck, Germany) | |
| sodium hydrogen carbonate | 44.00 mg |
| (CFS, Switzerland) | |
| poly(ethylene glycol) | 8.40 mg |
| (Lutrol^{(R)} E6000, BASF, Switzerland) | |
| sodium croscarmellose | 12.70 mg |
| (Ac-Di-Sol^{(R)}, FMC Corporation, USA) | |
| magnesium stearate | 1.48 mg |
| (Merck, Germany) | |
| colloidal silica | 0.60 mg |
| (Aerosil^{(R)} 200, Degussa AG, Hanau, Germany) | |

An effervescent granulate was prepared by means of a procedure similar to that described above in Example 2 under 2.a but for using the composition recited above. This mixture was then used in the compression procedure described below.

### 5.b Preparation of a granulate adapted to form the innermost intermediate layer

The required amount of granulate was prepared to allow the production of 2,000 innermost intermediate layers each having a weight of about 250 mg and the following composition:

| | |
|---|---|
| dibasic calcium phosphate | 125.00 mg |
| (Emcompress, Mendell, USA) | |
| glyceryl behenate | 100.00 mg |
| (Compritol^{(R)} 888 ATO, Gattefossé, France) | |
| polyvinylpyrrolidone | 21.25 mg |
| (Plasdone^{(R)} K29-32, ISP AG, Switzerland) | |
| magnesium stearate | 2.50 mg |
| (Merck, Germany) | |
| colloidal silica | 1.25 mg |
| (Aerosil^{(R)} 200, Degussa AG, Hanau, Germany) | |

The required amount of granulate was prepared by means of a procedure similar to that described above in Example 2 under 2.b but for using the composition recited above instead of the composition recited in Example 2 under 2.b. This mixture was then used in the compression procedure described below.

### 5.c Preparation of a granulate adapted to form the outermost intermediate layer

The required amount of granulate was prepared to allow the production of 2,000 outermost intermediate layers each having a weight of about 207.5 mg and the following composition:

| | |
|---|---|
| diltiazem HCl | 23.00 mg |
| citric acid | 91.20 mg |
| (Merck, Germany) | |
| sodium hydrogen carbonate | 60.80 mg |
| (CFS, Switzerland) | |
| poly(ethylene glycol) | 12.00 mg |
| (Lutrol^{(R)} E6000, BASF, Switzerland) | |
| sodium croscarmellose | 17.55 mg |
| (Ac-Di-Sol^{(R)}, FMC Corporation, USA) | |
| magnesium stearate | 2.05 mg |
| (Merck, Germany) | |
| colloidal silica | 0.90 mg |
| (Aerosil^{(R)} 200, Degussa AG, Hanau, Germany) | |

The required amount of granulate was prepared by means of a procedure similar to that described above in Example 2 under 2.b but for using the composition recited above instead of the composition recited in Example 2 under 2.b. This mixture was then used in the compression procedure described below.

### 5.d Preparation of a granulate adapted to form the external coating layer

The required amount of granulate was prepared to allow the production of 2,000 external coating layers each having a weight of about 350 mg and the following composition:

| | |
|---|---|
| dibasic calcium phosphate | 175.00 mg |
| (Emcompress, Mendell, USA) | |
| glyceryl behenate | 140.00 mg |
| (Compritol^{(R)} 888 ATO, Gattefossé, France) | |
| polyvinylpyrrolidone | 29.75 mg |
| (Plasdone^{(R)} K29-32, ISP AG, Switzerland) | |
| magnesium stearate | 3.50 mg |
| (Merck, Germany) | |
| colloidal silica | 1.75 mg |
| (Aerosil^{(R)} 200, Degussa AG, Hanau, Germany) | |

The required amount of granulate was prepared by means of a procedure similar to that described above in Example 2 under 2.b but for using the composition recited above instead of the composition recited in Example 2 under 2.b. This mixture was then used in the compression procedure described below.

### 5.e Preparation of the cores

The cores were prepared by means of a rotating press machine type Kilian RUD (Kilian & Co. GmbH, Germany) equipped with circular convex punches having a 6 mm diameter, operating on the granulate prepared as described above under 5.a.

### 5.f Application of the innermost intermediate layers onto the cores

The cores previously prepared as described above under 5.e each were press-coated with an intermediate layer as described above in Example 2 under 2.e, except that circular convex punches were used that had a diameter of 8 mm, the first loading hopper supplied 120 mg of granulate, and the second loading hopper supplied 130 mg of granulate.

### 5.g Application of the outermost intermediate layers onto the innermost intermediate layers

The half-finished tablet systems made up of cores previously press-coated with an innermost intermediate layer as described above under 5.f each were press-coated with an outermost intermediate layer as described above in Example 2 under 2.e, except that circular convex punches were used that had a diameter of 9 mm, the first loading hopper supplied 95 mg of granulate, and the second loading hopper supplied 112.5 mg of granulate.

### 5.h Application of the external coating layers onto the outermost intermediate layers

The half-finished tablet systems made up of cores previously press-coated with an outermost intermediate layer as described above under 5.g each were press-coated with an external coating layer as described above in Example 2 under 2.e, except that circular convex punches were used that had a diameter of 11 mm, the first loading hopper supplied 150 mg of granulate, and the second loading hopper supplied 200 mg of granulate.

### 5.i Release characteristics of the tablet systems (Fig. 9)

The release characteristics of the tablet systems prepared as described above under 5.a to 5.h were determined as described above in Example 1 under 1.e but for stirring at 150 rpm. The *in vitro* release of the active substance from the tablet systems was investigated for 3 sample tablet systems subjected to respective dissolution runs. The investigation results are illustrated in Fig. 9 as respective time profile diagrams. The characteristics that appear therefrom will be discussed below.

### INTERPRETATION OF THE TIME PROFILE DIAGRAM OF EXAMPLE 5

From the time profile diagram of Example 5 illustrated in Fig. 9 the following characteristics clearly appear:

A first no-release period can be observed that is due to the external coating layer, followed by a first release period in which the active substance originates from the outermost intermediate layer, followed in turn by a second no-release period that is due to the innermost intermediate layer, followed in turn by a second release period in which the active substance originates from the core.

The first no-release period appears as a well-defined time interval having a duration of about 3 hours, observed between the start of the experiment and the start of the first release.

The first release of active substance is observed to take place within a release period of less than one hour duration.

The second no-release period appears as a well-defined time interval observed between the end of the first release and the start of the second release, having a duration of more than 6 hours in each instance, more specifically, of about 6 hours for one sample and about 7 hours for the two other samples.

In each instance the second release of active substance is observed to take place within a release period of less than one hour duration.

Thus, the release of both the first and second active substances is observed to reach completion within about 10 to 11 hours after the start of the experiment.

### EXAMPLE 6

### Preparation of diltiazem HCl five-element tablet systems with a pressed core and press-coated layers

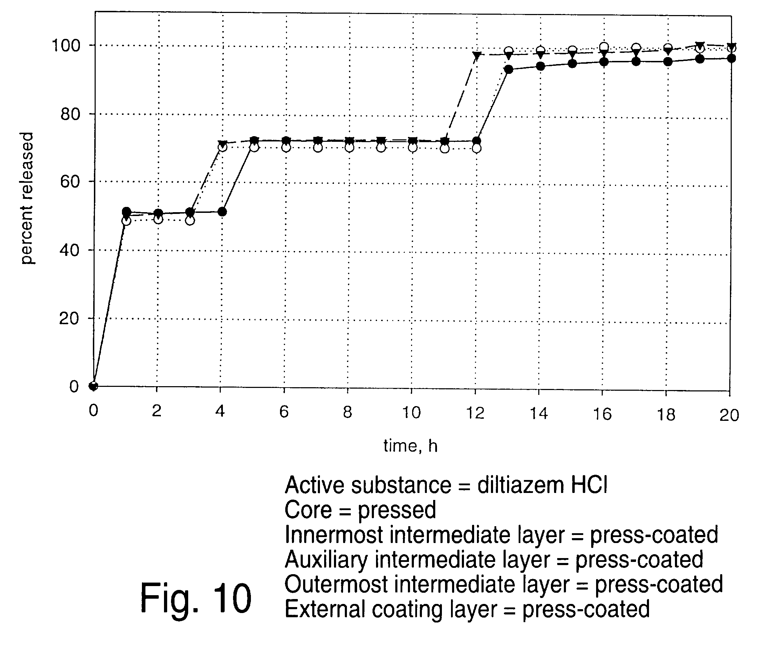

Tablets of the type illustrated in Fig. 4 were prepared, comprised of a core enclosed within an innermost intermediate layer itself enclosed within an auxiliary intermediate layer itself enclosed within an outermost intermediate layer itself enclosed within an external coating layer. The elements were produced by means of pressing the core and press-coating the layers thereon. Resulting release characteristics for these tablet systems are illustrated in Fig. 10 that will be described below.

### 6.a Preparation of a granulate adapted to form the core

The required amount of granulate had a composition and was prepared as described above in Example 5 under 5.a.

### 6.b Preparation of a granulate adapted to form the innermost intermediate layer

The required amount of granulate had a composition and was prepared as described above in Example 5 under 5.b.

### 6.c Preparation of a granulate adapted to form the auxiliary intermediate layer

The required amount of granulate had a composition and was prepared as described above in Example 5 under 5.c in respect of the outermost intermediate layer.

### 6.d Preparation of a granulate adapted to form the outermost intermediate layer

The required amount of granulate had a composition and was prepared as described above in Example 5 under 5.d in respect of the external coating layer.

### 6.e Preparation of a granulate adapted to form the external coating layer

The required amount of granulate was prepared to allow the production of 2,000 outermost intermediate layers each having a weight of about 355 mg and the following composition:

| | |
|---|---|
| diltiazem HCl | 40.00 mg |
| citric acid | 156.00 mg |
| (Merck, Germany) | |
| sodium hydrogen carbonate | 104.00 mg |
| (CFS, Switzerland) | |
| poly(ethylene glycol) | 20.00 mg |
| (Lutrol^{(R)} E6000, BASF, Switzerland) | |
| sodium croscarmellose | 30.00 mg |
| (Ac-Di-Sol^{(R)}, FMC Corporation, USA) | |
| magnesium stearate | 3.50 mg |
| (Merck, Germany) | |
| colloidal silica | 1.50 mg |
| (Aerosil^{(R)} 200, Degussa AG, Hanau, Germany) | |

The required amount of granulate was prepared by means of a procedure similar to that described above in Example 2 under 2.b but for using the composition recited above instead of the composition recited in Example 2 under 2.b. This mixture was then used in the compression procedure described below.

### 6.f Preparation of the cores

The cores were prepared as described above in Example 5 under 5.e.

### 6.g Application of the innermost intermediate layers onto the cores

The cores previously prepared as described above under 6.f each were press-coated with an innermost intermediate layer as described above in Example 5 under 5.f.

### 6.h Application of the auxiliary intermediate layers onto the innermost intermediate layers

The half-finished tablet systems made up of cores previously press-coated with an innermost intermediate layer as described above under 6.g each were press-coated with an auxiliary intermediate layer as described above in Example 5 under 5.g in respect of the outermost intermediate layer.

### 6.i Application of the outermost intermediate layers onto the auxiliary intermediate layers

The half-finished tablet systems made up of cores previously press-coated with an auxiliary intermediate layer as described above under 6.h each were press-coated with an outermost intermediate layer as described above in Example 5 under 5.h in respect of the outermost intermediate layer.

### 6.j Application of the external coating layers onto the outermost intermediate layers

The half-finished tablet systems made up of cores previously press-coated with an outermost intermediate layer as described above under 6.i each were press-coated with an external coating layer as described above in Example 2 under 2.e, except that circular convex punches were used that had a diameter of 12 mm, the first loading hopper supplied 160 mg of granulate, and the second loading hopper supplied 195 mg of granulate.

### 6.k Release characteristics of the tablet systems (Fig. 10)

The release characteristics of the tablet systems prepared as described above under 6.a to 6.j were determined as described above in Example 5 under 5.i. The *in vitro* release of the active substance from the tablet systems was investigated for 3 sample tablet systems subjected to respective dissolution runs. The investigation results are illustrated in Fig. 10 as respective time profile diagrams. The characteristics that appear therefrom will be discussed below.

### INTERPRETATION OF THE TIME PROFILE DIAGRAM OF EXAMPLE 6

A first release period can be observed in which the active substance originates from the external coating layer, followed by a first no-release period that is due to the outermost intermediate layer, followed in turn by a second release period in which the active substance originates from the auxililary intermediate layer, followed in turn by a second no-release period that is due to the innermost intermediate layer, followed in turn by a third release of active substance in which the active substance originates from the core.

The first and second no-release periods each appear as well-defined time intervals observed between the end of the immediately preceding and the start of the immediately following release of active substance, respectively.

The first, second and third release of active substance each are observed in each instance to take place within a respective release period of less than one hour duration.

The first no-release period appears to have a duration of about 2 to 3 hours, more specifically, of about 2 hours for two samples and about 3 hours for the third sample.

The second no-release period appears to have a duration of about 7 to 8 hours, more specifically, of about 7 hours for two samples and about 8 hours for third sample.

Thus, the release of all of the first, second and third active substances is observed to reach completion within about 11 to 12 hours after the start of the experiment.

### REMARK ON THE TIME PROFILE DIAGRAMS OF EXAMPLES 1, 5 AND 6

It is important to note that the measurements performed in the experiments of Examples 1, 5 and 6 illustrated in Figs. 5, 9 and 10 have only been made at time intervals of one hour each. Thus, whereas all that can be said from the respective diagrams is that the respective releases each were completed for each sample within one hour from their respective start, it is strongly believed that the releases would have been found in each instance to proceed much faster, would measurements have been made at shorter time intervals.

### ILLUSTRATION OF TABLET FRAGMENTATION

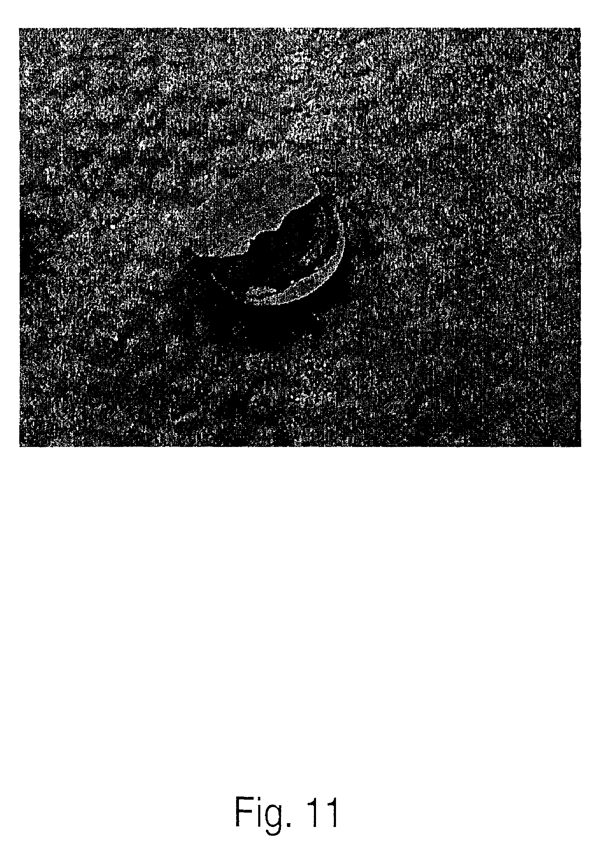

The fragmentation caused by dissolution of a tablet system according to the embodiment of Fig. 3 is depicted in the microphotography shown in Fig. 11 at the final stage of a dissolution run performed thereon. Thus, Fig. 11 also illustrates how fragmentation and dissolution proceeds.

In the instance illustrated, the tablet as manufactured was comprised of a core enclosed within an innermost intermediate layer itself enclosed within an outermost intermediate layer itself enclosed within an external coating layer. The core had been produced by pressing, both the innermost and outermost intermediate coating layer by spray-coating, and the external coating layer by press-coating. The microphotographic picture of Fig. 11 has been taken after complete release both of the first and second active substances, which means that the core and the outermost intermediate coating layer (which elements of the tablet contained active substance) both are totally dissolved and hence, have disappeared (as may be observed). The two other elements of the tablet (that do not contain active substance) are seen to have been forced open and appear, the external coating layer wide open like a gaping scallop, the innermost intermediate layer with a slit like a mussel about to open.

It can be readily understood from the picture of Fig. 11 that the external coating layer does not obstruct in any way the dissolution and/or outflow of substance from any of the underlying elements.

It can also be readily understood from the picture of Fig. 11 that the fast release of active substance from the core preferably is attained, using a core substrate whose effervescence will produce propellant gas capable of splitting open the remaining innermost intermediate layer and driving the active substance through the slit thus produced.

## Claims

1. A tablet system for pharmaceutical use capable, when contacted with an aqueous medium, of releasing at least one active substance during a release period of predetermined duration subsequent to a no-release period of predetermined duration,
the tablet system having a core-centred body comprised of at least one couple of tablet elements adjacent to each other, one of which is fully enclosed within the other one, thus to define an enclosing tablet element and an enclosed tablet element,
the enclosed element being comprised of active substance and formulated to release the active substance upon exposure to the aqueous medium while undergoing essentially complete degradation during the release period,
the enclosing element being devoid of active substance and formulated to delay the release of the active substance from the enclosed element by the no-release period,
**characterized in that**
the enclosing element is formulated to have an intrinsic porosity that remains substantially constant upon, and irrespective of, exposure of the enclosing element to the aqueous medium, said porosity allowing and controlling penetration of the aqueous medium, upon said exposure, through the enclosing element towards the enclosed element,
said penetration allowing the aqueous medium to eventually reach the enclosed element and then cause an alteration thereof at least at a surface thereof, which alteration causes the enclosing element to experience fragmentation, which fragmentation then allows the aqueous medium to directly reach the enclosed element to provide for rapid establishment and progress to completion of the release of active substance from the enclosed element.

2. A tablet system according to claim 1, wherein the degradation of the enclosed element will cause a swelling thereof that causes the enclosing element to become fragmented.

3. A tablet system according to claim 1, wherein the degradation of the enclosed element will cause an effervescence thereof that causes the enclosing element to become fragmented.

4. A tablet system according to claim 1, wherein the penetration of the aqueous medium through the enclosing element results in an increase of osmotic pressure within the enclosing element that causes the enclosing element to become fragmented.

5. A tablet system according to any of claims 1 to 4, wherein at least one tablet element of said couple of tablet elements is formed as a layer and made, using a layer-making procedure selected from film coating and pressing.

6. A tablet system according to claim 5, wherein the tablet elements of said couple of tablet elements both are formed as layers and made, using different layer-making procedures selected from film coating and pressing, respectively.

7. A tablet system according to any of claims 1 to 6, wherein the enclosing tablet element is selected to provide a corresponding no-release period that is longer than about 8 hours.

8. A tablet system according to any of claims 1 to 7 and comprised of a plurality of couples of tablet elements, the tablet system being made up of tablet elements adjacent to each other in succession and enclosing each other in succession from an innermost tablet element to an outermost tablet element over the whole plurality of couples, which will allow the tablet system to provide, upon exposure to the aqueous medium, a corresponding plurality of no-release and release periods subsequent to each other in alternate succession.

9. A method of making an enclosing element devoid of active substance as defined in claim 1 in a tablet system according to any of claims 1 to 8 in which the enclosing element is embodied as a film-coated layer, including the step of spray-coating a solution made of components of a film-coating composition dissolved in a pharmaceutically acceptable organic solvent, preferably in ethanol 96 % by volume with the remainder being water.
